Europäisches Patentamt
European Patent Office
Office européen des brevets

(11) Publication number: **0 341 837 A2**

(12) **EUROPEAN PATENT APPLICATION**

(21) Application number: 89303712.7

(51) Int. Cl.4: **C07D 239/48 , A61K 31/505**

(22) Date of filing: 14.04.89

Claims for the following Contracting States: ES + GR.

The title of the invention has been amended (Guidelines for Examination in the EPO, A-III, 7.3).

(30) Priority: **15.04.88 US 181983**
**15.04.88 US 181984**

(43) Date of publication of application:
**15.11.89 Bulletin 89/46**

(84) Designated Contracting States:
**AT BE CH DE ES FR GB GR IT LI LU NL SE**

(71) Applicant: **THE WELLCOME FOUNDATION LIMITED**
**183-193 Euston Road**
**London NW1 2BP(GB)**

(72) Inventor: **Kelley, James Leroy**
**10928 Raven Rock Drive**
**Raleigh North Carolina 27614(US)**
Inventor: **Bigham, Eric Cleveland**
**2511 Foxwood Drive**
**Chapel Hill North Carolina 27514(US)**

(74) Representative: **Rollins, Anthony John et al**
**Group Patents & Agreements The Wellcome Research Laboratories Langley Court**
**Beckenham Kent BR3 3BS(GB)**

(54) **Glutamic acid derivatives.**

(57) The present invention relates to Compounds of formula (I):

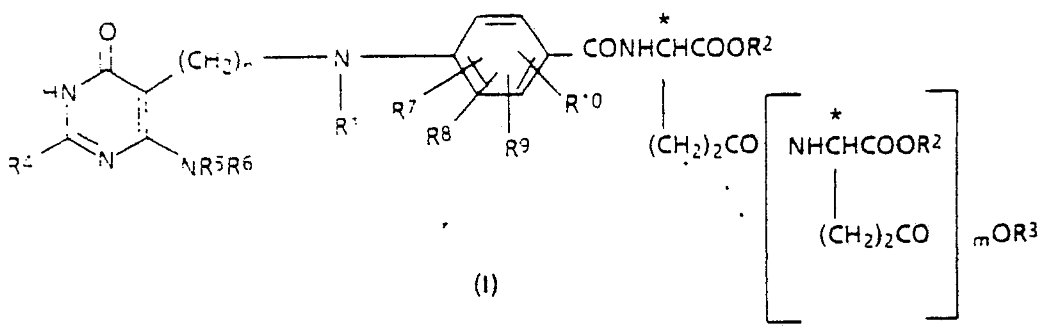

(I)

wherein $R^1$ is hydrogen;
$R^2$ and $R^3$ are the same or different and are each hydrogen or $C_{1-4}$ alkyl;
$R^4$ is $NR^{11}R^{12}$;
$R^5$, $R^6$, $R^{11}$ and $R^{12}$ are each hydrogen;
m is zero;
n is 3 and one of $R^7$, $R^8$, $R^9$ and $R^{10}$ is chloro, fluoro, methyl or methoxy and the rest are hydrogen;
or n is 4 and each of $R^7$, $R^8$, $R^9$ and $R^{10}$ is hydrogen;
and pharmaceutically acceptable salts thereof, methods for their preparation, pharmaceutical formulations containing them and their use in the treatment of tumours.

EP 0 341 837 A2

## NOVEL COMPOUNDS

The present invention relates to a group of novel glutamic acids, acid esters and salts, processes and intermediates for their preparation, pharmaceutical formulations containing them, and to their use in medicine and agriculture.

Our European Patent Application No 268377 describes a structurally distinct class of novel substituted glutamic acids and glutamic esters of the formula

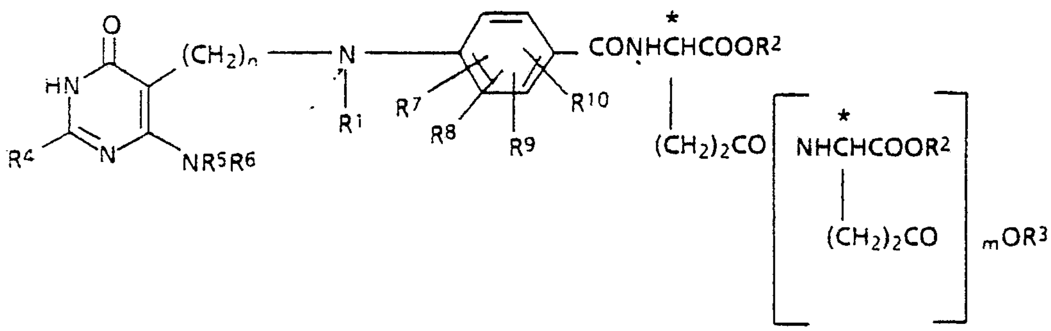

wherein $R^1$ is hydrogen, $C_{1-4}$ alkyl, acetyl or formyl; $R^2$ and $R^3$ are the same or different and are hydrogen or $C_{1-4}$ alkyl; $R^4$ is $NR^{11}R^{12}$; $R^{11}$, $R^{12}$, $R^5$ and $R^6$ are the same or different and are hydrogen, $C_{1-4}$ alkyl or $C_{1-12}$ acyl; $R^7$, $R^8$, $R^9$ and $R^{10}$ are the same or different and are hydrogen, halo, $C_{1-4}$ haloakyl, $C_{1-4}$ alkyl and $C_{1-4}$ alkoxy; and n is 2, 3, 4 or 5; m is 0 or an integer from 1 to 6; or a salt thereof.

These compounds have been found to possess anti-neoplastic activity in that they are able to inhibit the unregulated multiplication and proliferation of undifferentiated cells.

EPA 268377 was unpublished at the priority date of the present invention.

We have now found a further group of glutamic acids which also possess the above mentioned anti-neoplastic activity. Such activity has been demonstrated against human breast adenocarcinoma cells in the cell culture cytotoxicity test which is described hereinafter.

Accordingly, the present invention provides a compound of formula (I):

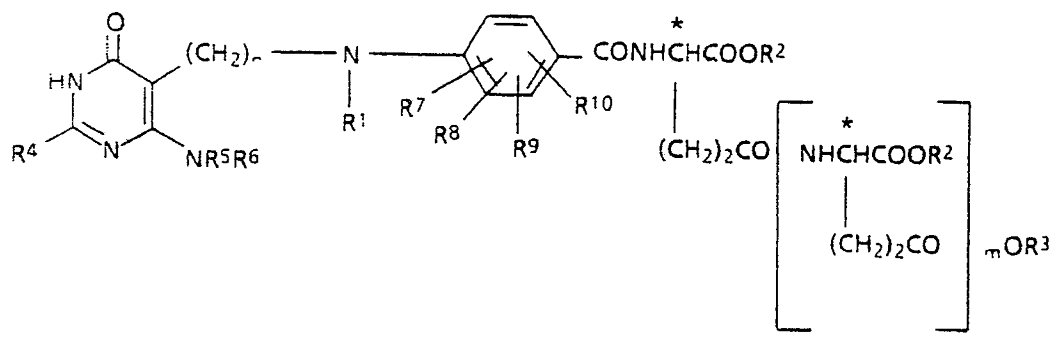

wherein $R^1$ is hydrogen;
$R^2$ and $R^3$ are the same or different and are each hydrogen or $C_{1-4}$ alkyl;
$R^4$ is $NR^{11}R^{12}$;
$R^5$ $R^6$, $R^{11}$ and $R^{12}$ are each hydrogen;
m is zero;
n is 3 and one of $R^7$, $R^8$, $R^9$ and $R^{10}$ is chloro, fluoro, methyl or methoxy and the rest are hydrogen;
or n is 4 and each of $R^7$, $R^8$ $R^9$ and $R^{10}$ is hydrogen;
and pharmaceutically acceptable salts thereof.

Examples of $R_2$ and $R_3$, when $C_{-4}$ alkyl, include methyl and ethyl. It is, however, preferred that $R_2$ and $R_3$ are both hydrogen.

The compounds of the present invention have an asymmetric carbon atom indicated by the asterisk in formula (I) and are, therefore, capable of existing as optical isomers. Although all such isomers, individually and as mixtures, are included within the scope of the present invention, the L-optical isomers are preferred.

In particular the present invention provides a compound selected from:
N-[4-(3-(2,4-diamino-1,6-dihydro-6-oxo-5-pyrimidinyl)propylamino)-2-fluorobenzoyl]-(L)-glutamic acid,
N-[4-(3-(2,4-diamino-1,6-dihydro-6-oxo-5-pyrimidinyl)propylamino)-3-fluorobenzoyl]-(L)-glutamic acid,
N-[4-(3-(2,4,-diamino-1,6-dihydro-6-oxo-5-pyrimidinyl)propylamino)-3-methylbenzoyl]-(L)-glutamic acid,
N-[4-(3-(2,4-diamino-1 ,6-dihydro-6-oxo-5-pyrimidinyl)propylamino)-3-(methoxy)benzoyl]-(L)-glutamic acid,
N-[4-(3-(2,4-diamino-1,6-dihydro-6-oxo-5-pyrimidinyl)propylamino)-2-chlorobenzoyl]-(L)-glutamic acid, and
N-[4[[4-(2,4-diamino-1,6-dihydro-6-oxo-5-pyrimidinyl)butyl]amino]benzoyl]-(L)-glutamic acid,
or a pharmaceutically acceptable salt thereof.

Of the compounds exemplified hereinafter, those that are preferred include the compound of Example 2.

As salts of the compounds of the present invention, there are included acid addition salts derived from either of the two terminal amino groups that substitute the pyrimidyl moiety or from the amino group present in the chain between the phenylene and the -(CH2)n- moieties and salts comprising an anionic species derived from a compound of formula (I), wherein one or both of $R_2$ and $R_3$ is or are hydrogen, and a cation. In both types of salts, the anti-neoplastic activity resides in the moiety derived from the compound of the invention as defined herein and the identity of the other component is of less importance although for therapeutic and prophylactic purposes it is, preferably, pharmaceutically acceptable to the patient. Examples of pharmaceutically acceptable acid addition salts include those derived from mineral acids, such as hydrochloric, hydrobromic, phosphoric, metaphosphoric, nitric and sulphuric acids, and organic acids, such as tartaric, acetic, citric, malic, lactic, fumaric, benzoic, glycollic, gluconic, succinic and arylsulphonic, for example p-toluenesulphonic, acids. Examples of salts comprising an anionic species derived from a compound of formula (I), wherein one or both of $R_2$ and $R_3$ is or are hydrogen, and a cation include ammonium salts, alkali metal salts, such as sodium and potassium salts, alkaline earth salts, such as magnesium and calcium salts, and salts formed with organic bases, for example, amino salts derived from mono-, di- or tri-(lower alkyl) or (lower alkanol)amines, such as triethanolamine and diethylaminoethylamine, and salts with heterocyclic amines such as piperidine, pyridine, piperazine and morpholine. The pharmaceutically acceptable salts together with the salts which are not thus acceptable have utility in the isolation and/or the purification of the compounds of the invention, and the pharmaceutically unacceptable salts are also useful in being convertible to the pharmaceutically acceptable salts by techniques well known in the art.

The present invention also provides a process for the preparation of a compound of formula (I), as defined herein, or a salt thereof, which comprises deacylating a compound of formula (II):

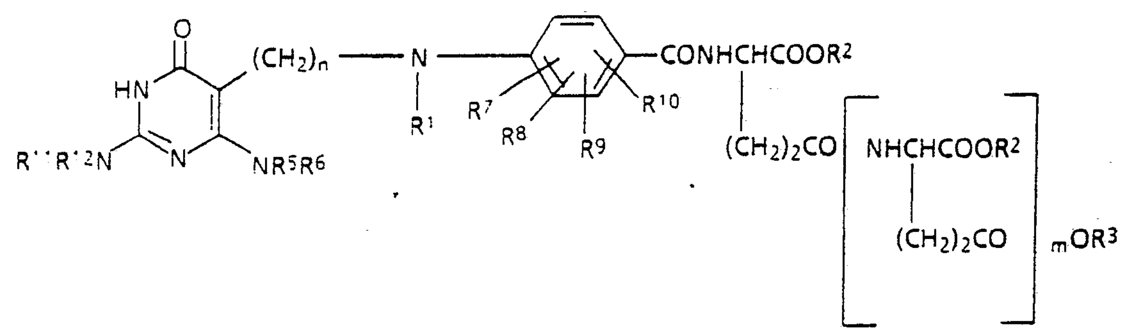

wherein $R_1$, $R_2$, $R_3$, $R_7$, $R_8$, $R_9$ and $R_{10}$ and n and m are as defined above in formula (I); one of the groups $R_5$, $R_6$, $R_{11}$ and $R_{12}$ is $C_{1-12}$ acyl, and the other groups $R_5$, $R_6$, $R_{11}$ and $R_{12}$ are the same or different and are hydrogen or $C_{1-12}$ acyl, and optionally thereafter converting one or both of $R_2$ and $R_3$, in the resulting compound of formula (I) into a different $R_2$, and/or $R_3$, and optionally forming a salt.

Preferably, $R_5$, $R_6$, $R_{11}$ and $R_{12}$, when $C_{1-12}$ acyl, are carboxylic $C_{1-12}$ acyl. Most preferably, $R_5$, $R_6$, $R_{11}$ and $R_{12}$, when $C_{1-12}$ acyl, are the same or different and are $C_{1-12}$ carboxylic acyl, in particular $C_{1-6}$ alkanoyl, especially acetyl.

The deacylation of a compound of formula (II) may be carried out conventionally. In the preferred case where $R_5$, $R_6$, $R_{11}$ and $R_{12}$, when $C_{1-12}$ acyl, are the same or different and are $C_{1-6}$ alkanoyl, the deacylation is, preferably, carried out at an elevated temperature in an alcoholic solvent, such as ethanol or ethanol containing a small amount of 2-mercaptoethanol so as to prevent any oxidation of the resulting compound of formula (I), in the presence of a base, such as sodium hydroxide. In the event that one or both of $R_2$ and $R_3$ in the compound of formula (II) is or are $C_{1-4}$ alkyl, then the conditions preferred for

deacylation are also likely to lead to deesterification. In such circumstances, the product of the deacylation is a compound of formula (I), wherein $R_2$ and $R_3$ are both hydrogen. In order, therefore, to prepare a compound of formula (I), wherein one or both of $R_2$ and $R_3$ is or are $C_{1-4}$ alkyl, the resulting compound of formula (I), wherein $R_2$ and $R_3$ are both hydrogen, is esterified as described hereinafter.

Examples of the optional conversion of $R_2$ and/or $R_3$ in the resulting compound of formula (I) into another $R_2$ and/or $R_3$ include the optional conversion of hydrogen into $C_{1-4}$ alkyl using conventional esterification reagents and conditions, for example a $C_{1-4}$ alkanol in the presence of an acid. This may be a useful optional conversion to carry out in the event that, in the preparation of a compound of formula (I), wherein one or both of $R_2$ and $R_3$ is or are $C_{1-4}$ alkyl, the compound undergoes deesterification as described hereinbefore. In such circumstances, the desired ester may be prepared from the free acid as just described.

The optional formation of a salt of a compound of formula (I) may be carried out conventionally.

The compounds of formula (II) may be prepared by reducing a mixture of a compound of formula (III):

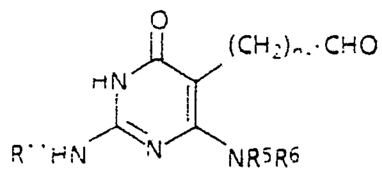

wherein $R_5$, $R_6$, $R_{11}$ and n are as defined herein, and a compound of formula (VI):

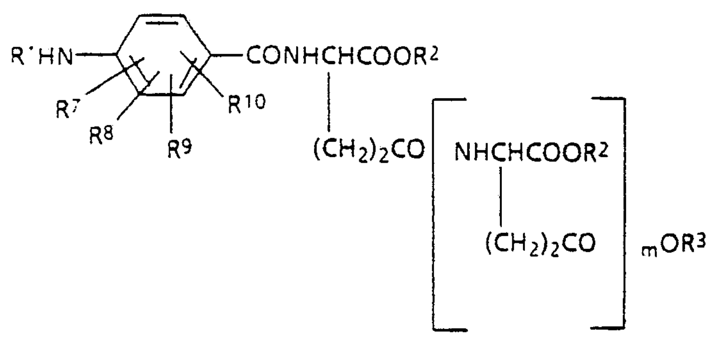

wherein $R_1$, $R_2$, $R_3$, $R_7$, $R_8$, $R_9$ and $R_{10}$ are as defined herein.

The reduction of a mixture of the compounds of formulae (III) and (VI) may be carried out in accordance with conventional reductive alkylation reactions. It is, however, preferred that the reduction is effected with sodium cyanoborohydride in a solvent in the presence of an acid, such as acetic acid which may also function as the solvent.

The compounds of formula (II), wherein n' is 3, may also, although less preferably, be prepared by reducing a mixture of a compound of formula (IV):

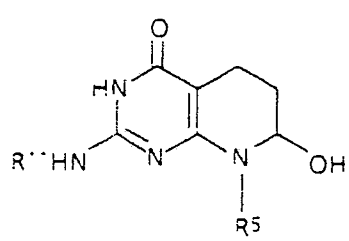

wherein $R_5$ and $R_{11}$ are as defined herein, and a compound of formula (VI) as defined herein.

The reduction of a mixture of the compounds of formulae (IV) and (VI) may be carried out analogously to the reduction of a mixture of the compounds of formulae (III) and (VI) in which sodium cyanoborohydride is used in the presence of an acid, such as acetic acid, to effect the reduction. It is, however, preferred, in the reduction of the mixture of the compounds of formulae (VI) and (IV), that acetic acid is not used also as the solvent but that, for example, methanol or a mixture of methanol and 2-methoxyethanol is used in this

regard. It is also preferred that the reduction is carried out in the presence of a dehydrating agent, such as molecular sieves.

The compounds of formulae (III) and (IV) may both be prepared by hydrolysis of a compound of formula (V):

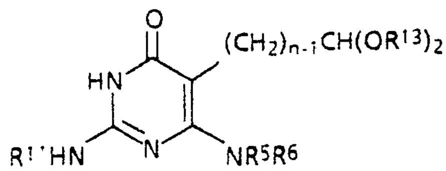

wherein $R_{11}$, $R_5$, $R_6$ and n are as defined herein and $R_{13}$ is $C_{1-4}$ alkyl, such as ethyl.

In the case of the preparation of a compound of formula (III), the hydrolysis may be carried out in a solvent, such as dichloromethane or acetone, in the presence of an acid, such as oxalic acid or 4-toluenesulphonic acid. In addition, in the case of the preparation of a compound of formula (III), wherein n is 3, the hydrolysis may also be carried out simply in water at room or a slightly elevated temperature.

In the case of the preparation of a compound of formula (IV), the hydrolysis is carried out with a compound of formula (V), wherein n is 3, and may be achieved simply in boiling or nearly boiling water.

The compound of formula (V) may be prepared by acylating a compound of formula (VII):

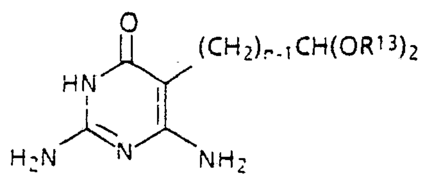

wherein $R_{13}$ and n are as defined herein.

The acylation of a compound of formula (VII) may be carried out conventionally using, for example, an acid anhydride in the presence of a base, such as pyridine.

The compound of formula (VII) may be prepared by reacting a compound of formula (VIII):

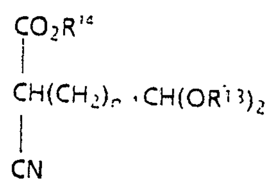

wherein $R_{13}$ and n are as defined herein and $R_{14}$ is $C_{1-4}$ alkyl, such as ethyl, and guanidine.

The reaction between the compound of formula (VIII) and guanidine may be carried out conventionally, for example, in a solvent, such as ethanol, in the presence of a base, such as sodium methoxide, at an elevated temperature.

The compound of formula (VIII) may be prepared by reacting a compound of formula (IX):

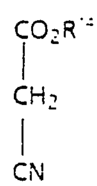

wherein $R_{14}$ is as defined herein, and a compound of formula (X):

$L-(CH_2)_{n-1}CH(OR^{13})_2$

wherein $R_{13}$ and n are as defined herein and L is a leaving group, such as chloro, bromo, iodo or tosyloxy.

The reaction between the compounds of formulae (IX) and (X) may be carried out conventionally, for example, in a solvent, such as ethanol, in the presence of a base, such as sodium methoxide.

The compounds of formulae (VI), (IX) and (X) are commercially available, or may be obtained by carrying out a published process for their preparation, or by carrying out a process analogous to a published process for the preparation of structurally analogous compounds. For example, the compounds of formula (VI) may be obtained by using the process described in J. Am. Chem. Soc., 1958, 80, page 5778 et seq.

While it is possible for the compounds or salts of the present invention to be administered as the raw chemical, it is preferred to present them in the form of a pharmaceutical formulation. Accordingly, the present invention further provides a pharmaceutial formulation, for medicinal application, which comprises a compound of the present invention or a pharmaceutically acceptable salt thereof, as hereinbefore defined, and a pharmaceutically acceptable carrier therefor.

The pharmaceutical formulation may optionally contain other therapeutic agents that may usefully be employed in conjunction with the compound or salt of the present invention, for example a dihydrofolate reductase inhibitor that is capable of enhancing the antineoplastic activity of the compounds and salts of the present invention. The expression "pharmaceutically acceptable" as used herein in relation to the carrier is used in the sense of being compatible with the compound or salt of the invention employed in the formulation and with any other therapeutic agent that may be present, and not being detrimental to the recipient thereof. The carrier itself may constitute one or more excipients conventionally used in the art of pharmacy that enable the compound or salt of the present invention and any other therapeutic agent that may be present, to be formulated as a pharmaceutical formulation.

The pharmaceutical formulations of the present invention include those suitable for oral, parenteral (including subcutaneous, intradermal, intramuscular and intravenous) and rectal administration although the most suitable route will probably depend upon, for example, the condition and identity of the recipient. The formulations may conveniently be presented in unit dosage form and may be prepared by any of the methods well known in the art of pharmacy. All methods include the step of bringing into association the active ingredient, i.e., the compound or salt of the present invention, with the carrier. In general, the formulations are prepared by uniformly and intimately bringing into association the active ingredient with a liquid carrier or, a finely divided solid carrier or both, and then, if necessary, forming the associated mixture into the desired formulation.

The pharmaceutical formulations of the present invention suitable for oral administration may be presented as discrete units, such as a capsule, cachet, tablet, or lozenge, each containing a predetermined amount of the active ingredient; as a powder or granules; as a solution or a suspension in an aqueous liquid or a non-aqueous liquid such as a syrup, elixir or a draught, or as an oil-in-water liquid emulsion or a water-in-oil liquid emulsion. The formulation may also be a bolus, electuary or paste.

Generally, a tablet is the most convenient pharmaceutical formulation suitable for oral administration. A tablet may be made by compressing or moulding the active ingredient with the pharmaceutically acceptable carrier. Compressed tablets may be prepared by compressing in a suitable machine the active ingredient in a free-flowing form, such as a powder or granules, in admixture with, for example, a binding agent, an inert diluent, a lubricating agent, a disintegrating agent and/or a surface active agent. Moulded tablets may be prepared by moulding in a suitable machine a mixture of the powdered active ingredient moistened with an inert liquid diluent. The tablets may optionally be coated or scored and may be formulated so as to provide slow or controlled release of the active ingredient.

The pharmaceutical formulations of the present invention suitable for parenteral administration include aqueous and non-aqueous sterile injection solutions which may contain, for example, an anti-oxidant, a buffer, a bacteriostat and a solution which renders the composition isotonic with the blood of the recipient, and aqueous and non-aqueous sterile suspensions which may contain, for example, a suspending agent and a thickening agent. The formulations may be presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions may be prepared from sterile powders, granules and tablets of the kind previously described.

The pharmaceutical formulations of the present invention suitable for rectal administration may be presented as a suppository containing, for example, cocoa butter and polyethylene glycol.

As mentioned hereinbefore, the compounds and salts of formula (I) have anti-neoplastic activity as demonstrated hereinafter in the human breast adenocarcinoma cell culture cytotoxicity tests, in which a representative number of the compounds of the present invention is shown to be active against particular cell lines. It has thus been established that the compounds of the present invention are able to inhibit

neoplastic growth. Therefore, the compounds and salts of the present invention are of use in medicine and in particular in the treatment of neoplastic growth, especially lymphocytic leukemia and malignant solid tumours such as melanoma, carcinoma and sarcoma in mammals. Accordingly, the present invention yet futher provides a method for the treatment of susceptible malignant tumors and leukemia in an animal, e.g., a mammal, which comprises administering to the animal a therapeutically effective amount of a compound or salt of the present invention. In the alternative, there is also provided a compound or salt of the present invention for use in medicine and in particular for use in the treatment of a neoplastic growth, e.g., leukemia and malignant tumors.

The present invention also provides the use of a compound of formula (I) or a salt thereof for the manufacture of a medicament for the treatment of neoplastic growth.

The animal requiring treatment with a compound or salt of the present invention is usually a mammal, such as a human being. Particular examples of a neoplastic growth requiring treatment include lymphocytic leukemia and malignant tumors.

As mentioned hereinbefore, the antineoplastic activity of the compounds and salts of the present invention may be enhanced by a dihydrofolate reductase inhibitor, for example, 2,4-diamino-6-(2,5-dimethoxybenzyl)-5-methylpyrido[2,3-d]pyrimidine hydrochloride. Therefore, it may be advantageous to employ with the compounds and salts of the present invention a dihydrofolate reductase inhibitor in the treatment of neoplastic growth.

The route by which the compound or salt of the present invention is administered to the animal may be oral, parenteral (including subcutaneous, intradermal, intramuscular, intravenous or rectal). If the compound or salt is presented in the form of a pharmaceutial formulation, which, as mentioned hereinbefore, is preferred, then the actual formulation employed will of course depend on the route of administration elected by the physician or veterinarian. For example, if oral administration is preferred, then the pharmaceutical formulation employed is, preferably, one which is suitable for such a route.

A therapeutically effective amount of a compound or salt of the present invention will depend upon a number of factors including, for example, the age and weight of the animal, the precise condition requiring treatment and its severity, the nature of the formulation, and the route of administration, and will ultimately be at the discretion of the attendant physician or veterinarian. However, an effective amount of a compound of the present invention for the treatment of neoplastic growth, in particular lymphocytic leukemia or a malignant tumor, will generally be in the range of 0.5 to 600 mg/kg body weight of recipient (mammal) per day and more usually in the range of 7.0 to 200 mg/kg body weight per day. Thus, for a 70 kg adult mammal, the actual amount per day would usually be from 490 to 14,000 mg and this amount may be given in a single dose per day or more usually in a number (such as two, three, four, five or six) of sub-doses per day such that the total daily dose is the same. An effective amount of a salt of the present invention may be determined as a proportion of the effective amount of the compound <u>per se</u>.

The treatment of neoplastic growth with a compound of the present invention may at times require the administration to the animal of an antidote or rescue agent. Particular examples of such agents include leucovorin, hypoxanthine and 5-aminoimidazole-4-carboxamide ribonucleotide (AICAR), although leucovorin is the most preferred.

The compounds of the invention may also be useful in the treatment or prophylaxis of citrus plants that are infected with the mycoplasma *Spiroplasma citri*. The compounds may be applied to the plants by methods known in the art, such as spraying, dusting, incorporating into the soil or injecting into the plant. The compounds may also be used in combatting certain bacterial organisms that are unable to synthesize their own folic acid and that, therefore, require preformed folate, such as *Lactobacillus casei* and *Streptococcus faecium*. The following examples and biological data are provided in illustration of the present invention and should not be construed as in any way constituting a limitation thereof.

## Example 1:Intermediates

### (a) Preparation of ethyl 2-cyano-5,5-diethoxypentanoate

To a stirred solution of 16.2 g (0.30 mol) of sodium methoxide in 100 ml of absolute ethanol was added 160 ml (170 g, 1.50 mol) of ethyl cyanoacetate. The solution was spin evaporated *in vacuo* at 40°C and the residual white solid was dissolved in 200 ml of dry dimethylformamide. To this solution was added 50 ml (50 g, 0.30 mol) of 3-chloropropionaldehyde diethyl acetal and a crystal of sodium iodide, and the solution was heated on a steam bath with magnetic stirring and protection from moisture for 5 hours. The red-brown

solution was cooled, poured into 300 ml of ice water and extracted with diethylether (6 x 200 ml). The organic phase was washed with water (3 x 50 ml), brine (50 ml) and dried over magnesium suphate. The solution was filtered, spin evaporated in vacuo, and the residue was distilled to give 41.3 g (57%) of a clear liquid, bp 90-108° (0.05 mm Hg) which was sufficiently pure for the next step. Fractional distillation gave as a main fraction a clear liquid, bp 103-108° (0.025 mm Hg); NMR (CDCl3) δ 1.20 (t, 6H, $CH(OCH_2CH_3)_2$), 1.31 (t, 3H, $CO_2CH_2CH_3$), 1.90 (m, 2H, $CH_2CHCN$), 3.60 (m, 5H, $CH(OCH_2CH_3)2$ + CHCN), 4.24 (q, 2H, $CO_2CH_2$), 4.51 (t, 1H, $CH(OCH_2)CH_3$); 1R (film) 2265, 1750, 1450 cm-1.

(b) Preparation of 3-(2,4-diamino-1,6-dihydro-6-oxo-5-pyrimidinyl)propionaldehyde diethyl acetal

To a solution of 22.0 g (407 mmol) of sodium methoxide in 400 ml of absolute ethanol was added 18.2 g (191 mmol) of guanidine hydrochloride and 46.0 g (189 mmol) of ethyl 2-cyano-5,5-diethoxypentanoate. The mixture was refluxed with stirring for 2.5 hours, stirred at ambient temperature overnight and then neutralized with 15 ml of acetic acid. The salts were removed by filtration and washed with 100 ml of ethanol. The combined filtrate and wash were spin evaporated in vacuo to give an off-white solid which was digested with ethyl acetate and cooled. The solid was collected and washed with ethyl acetate. This material, which contained sodium acetate, was dissolved in 150 ml of 1 N sodium hydroxide and then acidified with stirring to pH 5-6 with 10 ml of acetic acid. The resultant precipitate was collected, washed with 50 ml of cold water (product partly soluble in water) and dried; yield, 29.07 g (60%), mp 178-181°.

Recrystallization of a portion from ethyl acetate-ethanol gave the analytical sample, mp 177-178°; TLC ($C_6H_6$:EtOH/5:1); NMR (DMSO-d6) δ 1.11 (t, 6H, $CH_3$), 1.56 (m, 2H, $CH_2CH_2CH$), 2.19 (t, 2H, $CH_2CH_2CH$), 3.2-3.7 (m, 4H, 2 x $OCH_2$), 4.45 (t 1H H), (s, H, $NH_2$, 5.98 (s, 2H, NH2), 9.84 (s, 1H HNC(O)).

Elemental analysis: Calculated for $C_{11}H_{20}N_4O_3$: C, 51.6; H, 7.87; N, 21.9. Found: C, 51.7; H, 7.79; N, 21.7

(c) Preparation of 3-(2-acetylamino-4-diacetylamino-1,6-dihydro-6-oxo-5-pyrimidinyl) propionaldehyde diethyl acetal

A stirred mixture of 7.20 g (28.1 mmol) of 3-(2,4-diamino-1,6-dihydro-6-oxo-5-pyrimidinyl)-propionaldehyde diethyl acetal, 30 ml of dry pyridine and 30 ml of freshly distilled acetic anhydride was heated on an oil bath at 90° for 6 hours and then stirred at ambient temperature overnight. Solution occurred within 15 minutes as a mixture of diacetyl and triacetyl pyrimidinone formed; extended reaction was required to obtain only the title compound. The solution was spin evaporated in vacuo to give an oil. Ethyl acetate was added and spin evaporated several times until a solid was obtained. The solid was dispersed in cyclohexane and collected; yield, 8.88 g (82%), mp 126-137° (one spot on TLC). Recrystallization of a portion from cyclohexane-ethylacetate gave the analytical sample, mp 138-139°; TLC ($C_6H_6$:EtOH/5:1); NMR (DMSO-d6) δ 1.08 (t, 6H, $2CH_2CH_3$), 1.63 (m, 2H, $CH_2CH_2CH$), 2.13 (s, 3H, Ac), 2.27 (s, 6H, 2Ac), 2.2 (2H, $CH_2CH_2CH$, superimposed on the acetyl singlets), 3.2-3.7 (m, 4H, 2 x $OCH_2$), 4.42 (t, 1H, CH), 11.87 (br s, 2H, AcNH and HNC(O)).

Elemental analysis: Calculated for $C_{17}H_{26}N_4O_6$: C, 53.4; H, 6.85; N, 14.6. Found: C, 53.7; H, 6.87; N, 14.6.

(d) Route Via Compound of Formula (III): -Preparation of 3-(2-Acetylamino-4-diacetylamino-1,6-dihydro-6-oxo-5-pyrimidinyl)propionaldehyde

Method 1:

A solution of 3-(2-acetylamino-4-diacetylamino-1,6-dihydro-6-oxo-5-pyrimidinyl)propionaldehyde diethyl acetal (1.00 g, 2.6 mmol) in distilled water (30 ml) was stirred at room temperature for 18 hours. This solution was extracted with ethyl ether (3 x 50 ml), and chloroform 5 x 50 ml). The chloroform extracts were combined, washed with water (25 ml), brine (25 ml), dried ($MgSO_4$) and spin evaporated in vacuo. The residue was recrystallized from chloroform/cyclohexane to yield 0.200 g (25% of theory) of analytically pure 3-(2-acetylamino-4-diacetylamino-1,6-dihydro-6-oxo-5-pyrimidinyl)propionaldehyde quarter hydrate, mp 164-168°C.

Elemental analysis: Calc for $C_{13}H_{16}N_4O_5$ 1/4 H2O (MW 312.803): C, 49.9; H, 5.32; N, 17.9. Found:C, 49.8; H, 5.30; N, 17.6.

Method 2:

A solution of 3-(2-acetylamino-4-diacetylamino-1,6-dihydro-6-oxo-5-pyrimidinyl)propionaldehyde diethyl acetal (30.0 g, 78.5 mmol) in distilled water (600 ml) was stirred at 53°C for 3.5 hours and spin evaporated in vacuo at 45°C. The residue was dissolved in dichloromethane 750 ml), washed with brine (50 ml), dried ($MgSO_4$), and spin evaporated in vacuo to a dry solid.

Recrystallization from acetone gave 16.23 g (67% of theory) of 3-(2-acetylamino-4-diacetylamino-1,6-dihydro-6-oxo-5-pyrimidinyl)propionaldehyde, mp 164-168°C, which was identical to that prepared by Method 1.

Example 2: Preparation of N-[4-(3-(2,4-Diamino-1,6-dihydro-6-oxo-5-pyrimidinyl)propylamino)-2-fluorobenzoyl]-(L)-glutamate

(a) Preparation of di-tert-butyl N-(4-nitro-2-fluorobenzoyl)-(L)-glutamate

A solution of 2-fluoro-4-nitrobenzoic acid (6.26 g, 33.8 mmoles), (L)-glutamate di-tert-butyl ester hydrochloride (10.0 g, 33.8 mmoles), and 1-hydroxybenzotriazole (4.57 g, 33.8 mmoles) in dry dimethylformamide (150 mL) was stirred under nitrogen while triethylamine (3.42 g, 4.71 mL, 33.8 mmoles) was added in one portion. After 15 minutes, 1,3-dicyclohexylcarbodiimide (7.67 g, 37.2 mmoles) was added in portions during 1 minute. The reaction mixture was filtered after 20 hours and the solids rinsed with dichloromethane (1x50 mL). The filtrate was spin evaporated to an amber residue that was partitioned between dichloromethane and saturated bicarbonate solution (200 mL each). The organic phase was washed successively with saturated bicarbonate, 5% citric acid, and brine (1x200 mL each). The organic phase was then dried ($MgSO_4$), filtered, spin evaporated, and dried (high vacuum) to an oily yellow solid (11.9 g, 83%) that showed a major spot on tlc (silica, 2:1 hexane/ethyl acetate, $R_f = 0.5$). The product was purified by the flash chromatography technique to give a light green gum; yield, 9.7 g (67%); $^1$H-NMR ($CDCl_3$) δ 1.42 (s, 9H), 1.50 (s, 9H), 2.02-2.41 (m, 4H), 4.72 (m, 1H), 7.44 (br t, 1H), 8.0-8.3 (m, 3H); uv (MeOH) 260 nm max (11000), 225 min (4710), 215 sh (7680); mass spec (CI) m/e 427 (24%), 371 (20), 315 (100). Anal. Calcd. for $C_{20}H_{27}FN_2O_7$ ($M_r$ 426.44): C, 56.33; H, 6.38; H, 6.38; N, 6.57. Found: C, 56.39; H, 6.40; N, 6.57.

(b) Preparation of Di-tert-butyl N-(4-amino-2-fluorobenzoyl)-(L)-glutamate

A solution of di-tert-butyl-N (4-nitro-2-fluorobenzoyl)-(L)-glutamate (3.6 g, 8.4 mmoles) in 95% ethanol (100 mL) was mixed with 10% palladium on carbon (0.25 g) and placed on a Parr apparatus. The mixture was shaken under hydrogen atmosphere for 2 hours. The reaction mixture was filtered, spin evaporated, and dried to give a white solid, di-tert-butyl N-(4-amino-2-fluorobenzoyl)-(L)-glutamate yield, 3.0 g (91%); mp 91-95° C; $^1$H-NMR ($CDCl_3$) δ 1.41 (s, 9N), 1.48 (s, 9H), 2.0-2.4 (m, 4H), 2.8 (br s, 2H), 4.71 (m, 1H), 6.34 (d, 1H), 6.48 (d, 1H), 7.17 (q, 1H), 7.86 (t, 1H); uv (MeOH) 280 nm max (16800), 238 min (2760), 212 sh (12100); mass spec (CI) m/e 397 (21%), 341 (13), 285 (100). Anal. Calcd. for $C_{20}H_{29}FN_2O_5$ ($M_r$ 396.45): C, 60.59; N, 7.37; N, 7.07. Found: C, 60.33; N, 7.47; N, 6.99.

(c) Preparation of Di-tert-butyl N-(4-((3-(2-(Acetylamino)-4-(diacetylamino-1 ,6-dihydro-6-oxo-5-pyrimidinyl)-propyl)amino)-2-fluorobenzoyl-(L)-glutamate

Both di-tert-butyl N-(4-amino-2-fluorobenzoyl)-(L)-glutamate (1.29 g, 3.24 mmoles) and 3-(2-acetylamino-4-diacetylamino-1,6-dihydro-6-oxo-5-pyrimidinyl)propionaldehyde (1.00 g, 3.24 mmoles) were stirred in absolute ethanol (25 mL) under nitrogen while glacial acetic acid (1.5 mL) was added dropwise during 1 minute. After solution was achieved (1 hour), activated 3Å molecular sieves (5 mL) were added. After stirring 5 hours, sodium cyanoborohydride (0.20 g, 3.24 mmoles) was added in one portion. The reaction mixture was filtered after 18 hours and the solids were rinsed with ethanol (2x10 mL). The filtrate

was spin evaporated to a brown residue that was partitioned between ethyl acetate and water (50 mL each). The organic phase was washed (1x100 mL 1:1 water/brine), dried (MgSO4), and filtered to give a clear brown solution that showed a major spot on tlc (silica, 5% methanol/ethyl acetate, $R_f = 0.5$). The solution was spin evaporated onto silica gel (20 g) and the product eluted by the flash chromatography technique to give a white solid; yield, 1.28 g (57%); mp >120 °C (grad. dec.); $^1$H-NMR (DMSO-$d_6$) $\delta$ 1.36 and 1.39 (2 s, 18H), 1.62 (m, 2H), 1.8-2.4 (m, 6H), 2.09 (s, 3H), 2.19 (s, 6H), 2.98 (m, 2H), 4.31 (m, 1H), 6.25 (d, $J_o^{F,H}$ = 15 Hz, 1H), 6.38 (d, $J_o^{H,H}$ = 9Hz, 1H), 6.66 (br m, 1H), 7.44 (t, $J_o^{H,H}$ and $J_m^{F,H}$ = 9 Hz, 1H), 7.68 (t, 1H), 11-12 (v br s, 1H); uv (MeOH) 299 nm max (11900), 258 min (3250); mass spec (FAB) m/e 689 (25%), 430 (100), 388 (56), 277 (40).

Anal. Calcd. for $C_{33}H_{45}FN_6O_9 \bullet H_2O$ ($M_r$ 706.77): C, 56.08; H, 6.70; N, 11.89. Found: C, 56.15; H, 6.57; N, 11.59.

(d) Preparation of N-[4-(3-(2-4-Diamino-1,6-dihydro-6-oxo-5-pyrimidinyl)propylamino)-2-fluorobenzoyl]-(L)-glutamic acid

A solution of di-tert-butyl N-(4-((3-(2-(acetylamino)-4-(diacetylamino-1 ,6-dihydro-6-oxo-5-pyrimidinyl)-propyl)amino)-2-fluorobenzoyl)-(L)-glutamate (0.70 g, 1.0 mmol) in freshly distilled trifluoroacetic acid (10 mL) was stirred under nitrogen for 1.5 hours. The solution was spin evaporated and dried (high vacuum) to a light yellow residue that was mixed with 1.0 N sodium hydroxide (20 mL) and stirred under nitrogen at 55 °C for 18 hours. The reaction mixture was chilled (ice bath), neutralized by dropwise addition of glacial acetic acid, and then adjusted to pH 2.5 by dropwise addition of 6 N hydrochloric acid. After stirring cold for 2 hours, the resulting precipitate was filtered. The wet cake was recrystallized from dimethylformamide/water to give a cream solid; yield, 0.26 g (57%); mp 195-200 °C; $^1$H-NMR (DMSO-$d_6$) $\delta$ 1.55 (m, 2H), 1.8-2.1 (m, 2H), 2.1-2.3 (m, 4H), 2.98 (m, 2H), 4.36 (m, 1H), 5.75 (br s, 2H), 5.92 (br s, 2H), 6.27 (dxd, $J_o^{F,H}$ = 15 Hz, $J_m^{H,H}$ = 2 Hz, 1H), 6.39 (dxd, $J_o^{H,H}$ = 9 Hz, $J_m^{H,H}$ = 2 Hz, 1H), 6.56 (br m, 1H), 7.46 (t, $J_o^{H,H}$ and $J_m^{F,H}$ = 9 Hz, 1H), 7.68 (t, 1H), 9.8 (v br s, 1H), 12.4 (v br s, 2H); uv (0.1 N NaOH) 295 nm max (21400), 288 min (20900), 274 max (24400), 244 min (8230), 220 max (20400). Anal. Calcd. for $C_{19}H_{23}FN_6O_6 \bullet 0.7H_20 \bullet 0.3C_3N_7NO$ ($M_r$ 484.96): C, 49.29; N, 5.51; N, 18.20. Found: C, 49.24; H, 5.38; N, 18.09.

Example 3: Preparation of N-[4-(3-(2,4-Diamino-1,6-dihydro-6-oxo-5 pyrimidinyl)propylamino)-3-fluorobenzoyl]-(L)-glutamic acid

(a) Preparation of Diethyl N-(4-nitro-3-fluorobenzoyl)-(L)-glutamate

A solution of 3-fluoro-4-nitrobenzoic acid (6.00 g, 32.4 mmoles), (L)-glutamic acid diethyl ester hydrochloride (7.77 g, 32.4 mmoles), and 1-hydroxybenzotriazole (4.38 g, 32.4 mmoles) in dry dimethylformamide (150 mL) was stirred under nitrogen while triethylamine (4.52 mL, 3.28 g, 32.4 mmoles) was added dropwise during 1 minute. To the reaction mixture was added 1,3-dicyclohexylcarbodiimide (7.36 g, 35.7 mmoles) in portions during 1 minute. After 20 hours the reaction mixture was filtered and the solids rinsed with dichloromethane (1x50 mL). The filtrate was spin evaporated (high vacuum) to an amber residue that was partitioned between dichloromethane and saturated bicarbonate solution (200 mL each). The organic phase was washed with saturated bicarbonate solution, 5% citric acid, and brine (1x200 mL each, respectively). The organic phase was then dried ($MgSO_4$), filtered, spin evaporated, and dried (high vacuum) to give a yellow solid (7.6 g, 63%) that showed a major spot on tlc (silica, 2:1 hexane/ethyl acetate, Rf = 0.3). The product was purified by the flash chromatography technique (same solvent system) to give a light yellow solid; yield, 5.7 g (48%); mp 78-80 °C; $^1$H-NMR ($CDCl_3$) $\delta$ 1.25 and 1.31 (2t overlapped, 6H), 2.2-2.3 (m, 2H), 2.5-2.6 (m, 2H), 4.14 and 4.25 (2q overlapped, 4H), 4.71 (m, 1H), 7.58 (br d, 1H), 7.7-8.2 (m, 3H); uv (MeOH) 253 nm max (8000), 232 min (5890); mass spec (CI) m/e 371 (100%), 325 (33). Anal. Calcd. for $C_{16}H_{19}FN_2O_7$ (Mr 370.33): C, 51.89; H, 5.17; N, 7.56. Found: C, 51.98; H, 5.20; N, 7.55.

(b) Preparation of Diethyl N-(4-amino-3-fluorobenzoyl)-(L)-glutamate

A solution of diethyl N-(4-nitro-3-fluorobenzoyl)-(L)-glutamate (5.3 g, 14 mmoles) in 95% ethanol (150

mL) was mixed with 10% palladium on carbon (0.4 g) and placed on a Parr apparatus. The mixture was shaken under hydrogen atmosphere for 1.5 hours. The reaction mixture was filtered, spin evaporated, and dried to give a cream solid; yield, 4.7 g (98%); mp 92-97° C; $^1$H-NMR ($CDCl_3$) δ 1.22 and 1.29 (2t overlapped, 6H), 1.6 (br s, 2H), 2.1-2.5 (m, 4H) 4.10 and 4.22 (2q overlapped, 4H), 4.74 (m, 1H), 6.75 (t, 1H), 6.84 (d, 1H), 7.4-7.5 (m, 2H); uv (MeOH) 281 nm max (12000), 236 min (1480), 210 sh (10600); mass spec (Cl) m/e 341 (100%), 138 (64). Anal. Calcd. for $C_{16}H_{21}FN_2O_5$ ($M_r$ 340.35): C, 56.46; H, 6.22; N, 8.23. Found: C, 56.43; H, 6.27; N, 8.17.

(c) Preparation of Diethyl N-[4-(3-(2-acetylamino-4-diacetylamino-1,6-dihydro-6-oxo-5-pyrimidinyl)-propylamino)-3-fluorobenzoyl]-(L)-glutamate

Both diethyl N-(4-amino-3-fluorobenzoyl)-(L)-glutamate (2.21 g, 6.49 mmoles) and 3-(2-acetylamino-4-diacetylamino-1,6-dihydro-6-oxo-5-pyrimidinyl)propionaldehyde (2.00 g, 6.49 mmoles) were stirred in absolute ethanol (50 mL) under nitrogen while glacial acetic acid (3 mL) was added dropwise during 1 minute. After solution was achieved (1 hour), activated 3Å molecular sieves (10 mL) were added and stirring was continued 5 hours. The reaction mixture was chilled (ice bath) and sodium cyanoborohydride 408 mg, 6.49 mmoles) added in one portion. After stirring cold 0.5 hour, the bath was removed. The reaction mixture was allowed to warm to ambient temperature and stir 16 hours. The reaction mixture was filtered and the solids rinsed with ethanol (100 mL). The filtrate was spin evaporated to a brown residue that was partitioned between ethyl acetate and water (100 mL each). The organic phase was separated, washed (1x100 mL 1:1 water/brine), dried ($MgSO_4$), filtered, and spin evaporated to a small volume (20 mL). The solution was chromatographed on silica gel (175 g) using an ethyl acetate rinse (450 mL) followed by elution of the product with 5% added methanol. Like fractions were combined by spin evaporation and dried to give a white solid; yield, 2.66 g (65%); mp 76-84° C (dec); $^1$H-NMR (DMSO-$d_6$) δ 1.15 and 1.16 (2t overlapped, 6H), 1.64 (m, 2H), 2.00 (m, 2H), 2.11 (s, 3H), 2.20 (s, 6H), 2.27 (m, 2H), 2.40 (t, 2H), 3.10 (m, 2H), 3.9-4.1 (2q overlapped, 4H), 4.35 (m, 1H), 6.10 (m, 1H), 6.66 (t, $J_o^{H,H}$ and $J_m^{F,H}$ = 9 Hz, 1H), 7.5-7.6 (m, 2H), 8.35 (d, 1H), 11.9 (v br d, 1H); uv (MeOH) 299 nm max (28000), 257 min (7500); mass spec (FAB) m/e 633 (23%), 430 (100), 388 (87). Anal. Calcd. for $C_{29}H_{37}FN_6O_9$ ($M_r$ 632.64): C, 55.06; H, 5.90; N, 13.28. Found: C, 54.99; H, 5.93; N, 13.22.

(d) Preparation of N-[4-(3-(2,4-Diamino-1,6-dihydro-6-oxo-5-pyrimidinyl)propylamino)-3-fluorobenzoyl]-(L)-glutamic acid

A solution of diethyl N-[4-(3-(2-acetylamino-4-diacetylamino-1,6-dihydro-6-oxo-5-pyrimidinyl)-propylamino)-3-fluorobenzoyl]-(L)-glutamate (500 mg, 0.790 mmoles) in 1.0 N sodium hydroxide (50 mL) was stirred at 55° C under nitrogen for 18 hours. After cooling to room temperature, the reaction mixture was adjusted to pH 4.0 by the gradual dropwise addition of glacial acetic acid to precipitate a white solid. The slurry was chilled (ice bath) 1 hour before the solid was filtered, rinsed quickly with ethyl ether (1x15 mL), and dried to give a white solid (376 mg, >100%). A portion (200 mg) was recrystallized from dimethylformamide/water to give the analytical sample as a white solid; yield, 119 mg (60% recovery); mp >175° C (grad dec); $^1$H-NMR (DMSO-$d_6$) δ 1.56 (m, 2H), 1.8-2.1 (m, 2H), 2.1-2.4 (m, 4H), 3.07 (m, 2H), 4.34 (m, 1H), 5.78 (s, 2H), 5.93 (s, 2H), 6.24 (br s, 1H), 6.70 (t, $J_o^{H,H}$ and $J_m^{F,H}$ = 9 Hz, 1H), 7.55 and 7.56 (2d overlapped, $J_o^{F,H}$ = 12 Hz, $J_o^{H,H}$ = 9 Hz, 2H), 8.23 (d, 1H), 9.8 (v br s, 1H), 12.3 (v br s, 2H); uv (0.1 N NaOH) 295 nm max (19800), 287 min (19200), 273 max (23200), 242 min (8120), 220 max (18800). Anal. Calcd. for $C_{19}H_{23}FN_6O_6 \bullet 0.4H_2O \bullet 0.3C_3H_7NO$ ($M_r$ 479.56): C, 49.84; H, 5.44; N, 18.40. Found: C, 50.04; H, 5.38; N, 18.25.

Example 4 : Preparation of N-[4-(3-(2,4-Diamino-1,6-dihydro-6-oxo-5 pyrimidinyl)propylamino)-3-methylbenzoyl]-(L)-glutamic acid

(a) Preparation of Diethyl N-(4-amino-3-methylbenzoyl)-(L)-glutamate

A solution of diethyl N-(3-methyl-4-nitrobenzoyl)-(L)-glutamate (Cosulich, D.B. *et al., J. Am. Chem. Soc.*, **1953**, 75, 4675) (11 .0 g, 30.0 mmoles) in 95% ethanol (250 mL) was mixed with 10% palladium on

carbon (0.8 g) and placed on a Parr apparatus. The mixture was shaken under hydrogen atmosphere for 2 hours. The reaction mixture was filtered, spin evaporated, and dried to give a beige solid; yield, 10.1 g (100%); mp 79-82° C; $^1$H-NMR (DMSO-$d_6$) δ 1.15 and 1.16 (2t overlapped, 6H), 2.00 (m, 2H), 2.06 (s, 3H), 2.39 (t, 2H), 4.03 and 4.06 (2q overlapped, 4H), 4.33 (m, 1H), 5.40 (s, 2H), 6.56 (d, Jo = 8 Hz, 1H), 7.47 (d, Jo = 8 Hz, 1H), 7.50 (s, 1H), 8.16 (d, 1H); uv (MeOH) 284 nm max (17000), 241 min (3020); mass spec (Cl) m/e 337 (44%), 134 (100). Anal. Calcd. for $C_{17}H_{24}N_2O_5$ ($M_r$ 336.38): C, 60.70; H, 7.19; N, 8.33. Found: C, 60.76; H, 7.23; N, 8.31.

(b) Preparation of Diethyl N-[4-(3-(2-acetylamino-4-diacetylamino-1,6-dihydro-6-oxo-5-pyrimidinyl)-propylamino)-3-methylbenzoyl]-(L)-glutamate

Both diethyl N-(4-amino-3-methylbenzoyl)-(L)-glutmate (2.18 g, 6.49 mmoles) and 3-(2-acetylamino-4-diacetylamino-1,6-dihydro-6-oxo-5-pyrimidinyl)propionaldehyde (2.00 g, 6.49 mmoles) were stirred in absolute ethanol (50 mL) under nitrogen while glacial acetic acid (3 mL) was added dropwise during 1 minute. After solution was achieved (1 hour), activated 3Å molecular sieves (10 mL) were added and stirring was continued 5 hours. The reaction mixture was chilled (ice bath) and sodium cyanoborohydride (408 mg, 6.49 mmoles) added in one portion. After stirring cold 0.5 hour, the bath was removed and the reaction mixture allowed to warm to ambient temperature. The solids were filtered and rinsed with ethanol (100 mL) after 16 hours. The filtrate was spin evaporated to a residue that was partitioned between ethyl acetate and water (100 mL each). The organic phase was separated, washed (1x100 mL 1:1 water/brine), dried ($MgSO_4$), filtered, and spin evaporated to a small volume (15 mL). The solution was chromatographed on silica gel (175 g) using an ethyl acetate rinse (450 mL) followed by elution of the product with 5% added methanol. Like fractions were combined by spin evaporation and dried to give a clear semi-solid; yield, 1.45 g (36%); $^1$H-NMR (DMSO-$d_6$) δ 1.15 and 1.16 (2t overlapped, 6H), 1.66 (m, 2H), 2.00 (m, 2H), 2.08 (s, 3H), 2.11 (s, 3H), 2.19 (s, 6H), 2.29 (m, 2H), 2.39 (t, 2H), 3.11 (m, 2H), 3.9-4.1 (2q overlapped, 4H), 4.33 (m, 1H), 5.46 (m, 1H), 6.47 (d, Jo = 8 Hz, 1H), 7.53 (s, 1H), 7.57 (d, Jo = 8 Hz, 1H), 8.20 (d, 1H), 11.8 (v br s, 1H); uv (MeOH) 301 nm max (31000), 258 min (6050); mass spec (FAB) m/e 628 (8%), 426 (100), 384 (95). Anal. Calcd. for $C_{30}H_{40}N_6O_9$•0.3 $H_2O$•0.5 $C_4H_8O_2$ ($M_r$ 678.14): C, 56.68; H, 6.63; N, 12.39. Found: C, 56.61; H, 6.70; N, 12.46.

(c) Preparation of N-[4-(3-(2,4-Diamino-1,6-dihydro-6-oxo-5-pyrimidinyl)propylamino)-3-methylbenzoyl]-(L)-glutamic acid

A solution of diethyl N-[4-(3-(2-acetylamino-4-diacetylamino-1,6-dihydro-6-oxo-5-pyrimidinyl)-propylamino)-3-methylbenzoyl]-(L)-glutamate (0.70 g, 1.1 mmoles) in 1.0 N sodium hydroxide (20 mL) was stirred at 55° C under nitrogen for 18 hours. The reaction mixture was chilled (ice bath) and adjusted to pH 2.5 by the gradual dropwise addition of 6.0 N hydrochloric acid. After stirring cold 2 hours, the resulting precipitate was filtered. An attempted recrystallization of the wet cake from dimethylformamide/water failed, so the solution was spin evaporated to a clear oil. The oil was mixed with pH 2.1 aqueous HCl (20 mL) and sonicated 5 minutes to obtain a cream solid which was filtered and dried to give the product; yield, 0.12 g (24%); mp 181° C (eff.); $^1$H-NMR (DMSO-$d_6$) δ 1.58 (m, 2H), 1.8-2.1 (m, 2H), 2.12 (s, 3H), 2.2-2.4 (m, 4H), 3.08 (m, 2H), 4.34 (m, 1H), 5.78 (br s, 2H), 5.94 (br s, 2H), 6.52 (d, Jo = 9 Hz, 1H), 7.53 (s overlapped, 1H), 7.57 (d overlapped, Jo = 10 Hz, 1H), 8.06 (d, 1H), 10.0 (v br s, 1H), 12.2 (v br s, 2H); uv (0.1 N NaOH) 296 nm max (18000), 286 min (17500), 274 max (19900), 247 min (5890), 215 max (21000). Anal. Calcd. for $C_{20}H_{26}N_6O_6$•$H_2O$•0.2$C_3H_7NO$ ($M_r$ 479.10): C, 51.64; H, 6.19; N, 18.13. Found: C, 51.63; H, 6.07; N, 18.07.

Example 5 : Preparation of N-[4-(3-(2,4-Diamino-1,6-dihydro-6-oxo-5-pyrimidinyl)propylamino)-3-(methoxy)-benzoyl]-(L)-glutamic acid

(a) Preparation of Diethyl N-(3-methoxy-4-nitrobenzoyl)-(L)-glutamate

A solution of 3-methoxy-4-nitrobenzoic acid (10.0 g, 50.7 mmoles), (L) glutamate diethyl ester hydrochloride (12.2 g, 50.7 mmoles), and 1-hydroxybenzotriazole (6.85 g, 50.7 mmoles) in dry dimethylformamide (250 mL) was stirred under nitrogen while triethylamine (5.13 g, 7.07 mL, 50.7 mmol) was added

dropwise during 1 minute. After 15 minutes, 1,3-dicyclohexylcarbodiimide (11.5 g, 55.8 mmoles) was added in portions during 2 minutes. The reaction mixture was filtered after 18 hours and the solids were rinsed with dichloromethane (2x50 mL). The filtrate was spin evaporated and dried to an amber residue that was dissolved in dichloromethane (300 mL). The organic phase was washed successively with saturated bicarbonate solution, 5% citric acid, and 1:1 brine/water (1x300 mL each). The organic phase was then dried ($MgSO_4$) and filtered to give a brown-orange solution that showed a major spot on tlc (silica, 2:1 hexane/ethyl acetate, $R_f$=0.2). The solution was poured onto a silica gel column (500 g, pre-wet with dichloromethane) and rinsed with dichloromethane (500 mL). The product was removed by gradient elution using 10% and 15% added ethyl acetate (8x250 mL fractions each). Like fractions were combined by spin evaporation and dried (high vacuum) to give a clear yellow oil; yield, 11.5 g (59%), $^1$H-NMR (DMSO-$d_6$) $\delta$ 1.15 and 1.18 (2t overlapped, 6H), 1.9-2.2 (m, 2H), 2.45 (t obscured by DMSO, 2H), 3.98 (s, 3H), 3.9-4.2 (2q overlapped, 4H), 4.46 (m, 1H), 7.58 (dxd, $J_o$ = 8 Hz, $J_m$ = 2 Hz, 1H), 7.71 (d, $J_m$ = 2 Hz, 1H), 7.97 (d, $J_o$ = 8 Hz, 1H), 9.00 (d, 1H); uv (MeOH) 327 nm max (2940), 295 min (2090), 257 sh (6640), 235 sh (9140), 203 max (29700); mass spec (CI) m/e 383 (100%). Anal. Calcd. for $C_{17}H_{22}N_2O_8$ ($M_r$ 382.37): C, 53.40; N, 5.80; N, 7.33. Found: C, 53.29; H, 5.85; N, 7.29.

(b) Preparation of Diethyl N-(4-amino-3-methoxybenzoyl)-(L)-glutamate

A solution of diethyl N-(3-methoxy-4-nitrobenzoyl)-(L)-glutamate (11.0 g, 28.8 mmoles) in 95% ethanol (200 mL) was mixed with 10% palladium on carbon (0.8 g) and placed on a Parr apparatus. The mixture was shaken under hydrogen atmosphere for 1 hour. The reaction mixture was filtered, spin evaporated, and dried to give a clear viscous oil which on long-standing hardened to a white wax; yield, 9.95 g (99%); $^1$H-NMR (DMSO-$d_6$) $\delta$ 1.15 and 1.17 (2t overlapped, 6H), 1.8-2.2 (m, 2H), 2.40 (t, 2H), 3.79 (s, 3H), 4.0-4.2 (2q overlapped, 4H), 4.36 (m, 1H), 5.29 (br s, 2H), 6.60 (d, $J_o$ = 8 Hz, 1H), 7.3-7.4 (m, 2H), 8.25 (d, 1H); uv (MeOH) 301 nm max (17800), 280 sh (15700), 266 min (12600), 263 max (13200), 256 sh (12500), 245 min (8630), 204 max (40900); mass spec (CI) m/e 353 (56%), 150 (100). Anal. Calcd. for $C_{17}H_{24}N_2O_6 \bullet 0.1H_2O \bullet 0.3C_2H_6O$ ($M_r$ 368.01): C, 57.44; H, 7.12; N, 7.61. Found: C, 57.22; H, 6.95; N, 7.83.

(c) Preparation of Diethyl N-[4-(3-(2-acetylamino-4-diacetylamino-1,6-dihydro-6-oxo-5-pyrimidinyl)-propylamino)-3-methoxybenzoyl]-(L)-glutamate

Both diethyl N-(4-amino-3-methoxybenzoyl)-(L)-glutamate (3.52 g, 9.99 mmoles) and 3-(2-acetylamino-4-diacetylamino-1,6-dihydro-6-oxo-5-pyrimidinyl)propionaldehyde (3.08 g, 9.99 mmoles) were stirred in absolute ethanol (100 mL) under nitrogen while glacial acetic acid (6 mL) was added dropwise during 1 minute. After solution was achieved (0.5 hour), activated 3Å molecular sieves (20 mL) were added and stirring was continued 2 hours. The reaction mixture was chilled (ice bath) and sodium cyanoborohydride (628 mg, 9.99 mmoles) added in one portion. After stirring cold 0.5 hour, the bath was removed and the reaction mixture allowed to warm to ambient temperature. The reaction mixture was filtered after 16 hours and the solids were rinsed with ethanol (100 mL). The filtrate was spin evaporated and dried to a cream foam that was dissolved in ethyl acetate (100 mL). The organic phase was washed (2x100 mL 1:1 brine/water) and poured onto a silica gel column (175 g, pre-wet with ethyl acetate). The column was rinsed with ethyl acetate (400 mL) and then the product eluted with 5% added methanol. Like fractions were combined by spin evaporation and dried to give a white solid; yield, 2.07 g (32%); mp 73-84 °C (eff.); 1H-NMR (DMSO-d6) $\delta$ 1.15 and 1.17 (2t overlapped, 6H), 1.65 (br m, 2H), 1.9-2.3 (br m, 4H), 2.12 (s, 3H), 2.20 (s, 6H), 2.40 (t, 2H), 3.10 (br m, 2H), 3.80 (s, 3H), 4.0-4.2 (2q overlapped, 4H), 4.39 (m, 1H), 5.46 (br t, 1H), 6.47 (d, Jo = 8 Hz, 1H), 7.29 (d, Jm = 2 Hz, 1H), 7.42 (dxd, Jo = 8 Hz, Jm = 2 Hz, 1H), 8.28 (d, 1H), 11.9 (v br s, 2H); uv (MeOH) 306 nm max (25800), 258 min (3940), 227 sh (17800), 205 max (32400); mass spec (FAB) m/e 645.6 (62%), 442.5 (81), 400.1 (100). Anal. Calcd. for $C_{30}H_{40}N_6O_{10}$ ($M_r$ 644.68): C, 55.89; H, 6.25; N, 13.04. Found: C, 55.73; N, 6.33; N, 12.94.

(d) Preparation of N-[4-(3-(2,4-Diamino-1,6-dihydro-6-oxo-5-pyrimidinyl)propylamino)-3-methoxybenzoyl]-(L)-glutamic acid

A solution of diethyl N-[4-(3-(2-acetylamino-4-diacetylamino-1,6-dihydro-6-oxo-5-pyrimidinyl)-propylamino)-3-methoxybenzoyl]-(L)-glutamate (1.00 g, 1.55 mmoles) in 1.0 N sodium hydroxide (25 mL)

was stirred at 55° C under nitrogen for 18 hours. The reaction mixture was chilled (ice bath) and adjusted to pH 2.5 by the gradual dropwise addition of 6 N hydrochloric acid. After stirring cold 0.5 hour, the resulting precipitate was filtered, rinsed with pH 2.1 aqueous hydrochloric acid (2x25 mL), and dried to give a white solid; yield, 596 mg (83%); mp 165-184° C (grad. dec.); $^1$H-NMR (DMSO-$d_6$) δ 1.56 (m, 2H), 1.8-2.2 (br m, 2H), 2.2-2.4 (m, 4H), 3.07 (m, 2H), 3.81 (s, 3H), 4.37 (m, 1H), 5.6 (br s, 1H), 5.74 (s, 2H), 5.93 (s, 2H), 6.49 (d, $J_o$ = 8 Hz, 1H), 7.29 (d, $J_m$ = 2 Hz, 1H), 7.41 (dxd, $J_o$ = 8 Hz, $J_m$ = 2 Hz, 1H), 8.15 (d, 1H), 9.8 (v br s, 1H), 12.3 (v br s, 2H); uv (0.1 N NaOH) 306 nm max (15100), 287 min (11700), 273 max (15200), 250 min (6500), 215 max (25800). Anal. Calcd. for $C_{20}H_{26}N_6O_7$•$0.7H_2O$ ($M_r$ 475.07): C, 50.56; H, 5.81; N, 17.69. Found: C, 50.58; H, 5.85; N, 17.63.

Example 6 : Preparation of N-[4-(3-(2,4-Diamino-1,6-dihydro-6-oxo-5-pyrimidinyl)propylamino)-2-chlorobenzoyl]-(L)-glutamic acid

(a) Preparation of Diethyl N-(4-nitro-2-chlorobenzoyl)-(L)-glutamate

A solution of 2-chloro-4-nitrobenzoic acid (10.0 g, 49.6 mmoles), (L)-glutamate diethyl ester hydrochloride (11.9 g, 49.6 mmoles), and 1-hydroxybenzotriazole (6.7 g, 49.6 mmoles) in dry dimethylformamide (250 mL) was stirred under nitrogen while triethylamine (5.02 g, 6.92 mL, 49.6 mmoles) was added dropwise during 1 minute. After 15 minutes, 1,3-dicyclohexylcarbodiimide (11.3 g, 54.6 mmoles) was added in portions during 1 minute. The reaction mixture was filtered after 18 hours and the solids were rinsed with dichloromethane (2x50 mL). The filtrate was spin evaporated (high vacuum) to an amber residue that was dissolved in dichloromethane (300 mL). The organic phase was washed successively with saturated bicarbonate, 5% citric acid, and brine solutions (1x300 mL each). The organic phase was then dried ($MgSO_4$), filtered, and poured onto a silica gel column (500 g, pre-wet with dichloromethane). The column was rinsed with dichloromethane (500 mL), followed by gradient elution of the product with 10% and 15% added ethyl acetate (8x250 mL fractions each). Like fractions were combined by spin evaporation and dried to give a white solid; yield, 9.8 g (51%); mp 95-96° C; $^1$H-NMR (DMSO-$d_6$) δ 1.17 and 1.21 (2t overlapped, 6H), 1.8-2.2 (m, 2H), 2.46 (t obscured by DMSO, 2H), 4.0-4.2 (2q overlapped, 4H), 4.46 (m, 1H), 7.68 (d, $J_o$ = 8 Hz, 1H), 8.25 (dxd, $J_o$ = 8 Hz, $J_m$ = 2 Hz, 1H), 8.34 (d, $J_m$ = 2 Hz, 1H), 9.14 (d, 1H); uv (MeOH) 260 nm max (9930), 236 min (6180), 205 max (25600); mass spec (CI) m/e 387 (100%), 341 (40), 184 (21). Anal. Calcd. for $C_{16}H_{19}ClN_2O_7$ ($M_r$ 386.78): C, 49.68; H, 4.95; N, 7.24; Cl, 9.17. Found: C, 49.71; H, 4.98; N, 7.23; Cl, 9.23.

(b) Preparation of Diethyl N-(4-amino-2-chlorobenzoyl)-(L)-glutamate

A solution of diethyl N-(4-nitro-2-chlorobenzoyl)-(L)-glutamate (4.00 g, 10.3 mmoles) in glacial acetic acid (200 mL) was stirred under nitrogen while zinc dust (10.0 g) was added in one portion. After 1.5 hours the reaction mixture was filtered and the solids were rinsed with glacial acetic acid (2x25 mL). The filtrate was spin evaporated (high vacuum) to a clear yellow residue that was dissolved in dichloromethane (200 mL). The organic phase was washed with saturated bicarbonate solution (1x200 mL), dried (MgSO4), filtered, spin evaporated, and dried to give a yellow oil (3.69 g, 100%) that showed a major spot on tlc (silica, 4:1 dichloromethane/ethyl acetate, $R_f$ = 0.3). The product was purified by the flash chromatography technique to give a light yellow wax; yield, 3.12 g (85%); $^1$H-NMR (DMSO-$d_6$) δ 1.16 and 1.18 (2t overlapped, 6H), 1.8-2.1 (br m, 2H), 2.41 (t, 2H), 4.0-4.1 (2q overlapped, 4H), 4.33 (m, 1H), 5.72 (br s, 2H), 6.48 (dxd, $J_o$ = 8 Hz, $J_m$ = 2 Hz, 1H), 6.57 (d, $J_m$ = 2 Hz, 1H), 7.16 (d, $J_o$ = 8 Hz, 1H), 8.35 (d, 1H); uv (MeOH) 270 nm max (12600), 238 min (4500), 208 max (28800); mass spec (CI) m/e 357 (22%), 154 (100). Anal. Calcd. for $C_{16}H_{21}ClN_2O_5$ ($M_r$ 356.80): C, 53.86; H, 5.93; N, 7.85; Cl, 9.94. Found: C, 53.93; H, 5.95; N, 7.83; Cl, 10.03.

(c) Preparation of Diethyl N-[4-(3-(2-acetylamino-4-diacetylamino-1,6-dihydro-6-oxo-5-pyrimidinyl)-propylamino-2-chlorobenzoyl]-(L)-glutamate

Both diethyl-N-(4-amino-2-chlorobenzoyl-(L)-glutamate (2.84 g, 7.96 mmoles) and 3-(2-acetylamino-4-diacetylamino-1,6-dihydro-6-oxo-5-pyrimidinyl)propionaldehyde (2.70 g, 8.76 mmoles) were stirred in absolute ethanol (100 mL) under nitrogen while glacial acetic acid (6 mL) was added dropwise during 1 minute. After solution was achieved (0.5 hour), activated 3Å molecular sieves (20 mL) were added and stirring was continued 3 hours. The reaction mixture was chilled (ice bath) and sodium cyanoborohydride (500 mg, 7.96 mmoles) added in one portion. After stirring cold 0.5 hour, the bath was removed and the reaction mixture allowed to warm to ambient temperature. The reaction mixture was filtered after 16 hours and the solids were rinsed with ethanol (100 mL). The filtrate was spin evaporated and dried to a cream foam that was dissolved in ethyl acetate (100 mL). The organic phase was washed with 1:1 brine/water (2x100 mL) and then poured onto a silica gel column (175 g, pre-wet with ethyl acetate). The column was rinsed with ethyl acetate (400 mL) and the product was eluted with 5% added methanol. Like fractions were combined by spin evaporation to give a white solid; yield, 2.28 g (44%); mp 63-72°C (eff); $^1$H-NMR (DMSO-$d_6$) δ 1.16 and 1.18 (2t overlapped, 6H), 1.62 (m, 2H), 1.8-2.2 (br m, 2H), 2.12 (s, 3H), 2.23 (s, 6H), 2.26 (m, 2H), 2.42 (t, 2H), 2.99 (m, 2H), 3.9-4.2 (2q overlapped, 4H), 4.34 (m, 1H), 6.29 (br t, 1H), 6.4-6.6 (m, 2H), 7.21 (d, $J_o$ = 8 Hz, 1H), 8.35 (d, 1H), 11.9 (v br d, 2H); uv (MeOH) 289 nm max (26900), 257 min (13400), 207 max (44300); mass spec (FAB) m/e 649 (48%), 615 (22), 446 (100), 412 (46). Anal. Calcd. for $C_{29}H_{37}ClN_6O_9$ ($M_r$ 649.09): C, 53.66; H, 5.75; N, 12.95; Cl, 5.46. Found: C, 53.53; H, 5.80; N, 12.91; Cl, 5.42.

(d) Preparation of N-[4-(3-(2,4-Diamino-1,6-dihydro-6-oxo-5•pyrimidinyl)propylamino)-2-(chloro)benzoyl]-(L)-glutamic acid

A solution of diethyl N-[4-(3-(2-acetylamino-4-diacetylamino-1,6-dihydro-6-oxo-5-pyrimidinyl)-propylamino-2-chlorobenzoyl]-(L)-glutamate (1.00 g, 1.54 mmoles) in 1.0 N sodium hydroxide (25 mL) was stirred at 55°C under nitrogen for 18 hours. The reaction mixture was chilled (ice bath) and adjusted to pH 2.5 by the gradual dropwise addition of 6 N hydrochloric acid. After stirring cold 1 hour, the resulting precipitate was filtered, rinsed with cold pH 2.5 aqueous hydrochloric acid (2x25 mL), and dried to give a white solid; yield, 437 mg (61%); mp 150-178°C (eff); $^1$H-NMR (DMSO-$d_6$) δ 1.55 (m, 2H), 1.8-2.1 (m, 2H), 2.1-2.4 (m, 4H), 2.97 (m, 2H), 4.32 (m, 1H), 5.6-6.6 (v br s, 1H), 5.87 and 6.12 (2 br s, 4H), 6.50 and 6.52 (d and s overlapped, 2H), 7.23 (d, $J_o$ = 8 Hz, 1H), 8.20 (d, 1H), 10.0-12.6 (v br s, 1H); uv (0.1 N NaOH) 271 nm max (23000), 241 min (10000), 217 max (28000). Anal. Calcd. for $C_{19}N_{23}ClN_6O_6.1.3H_2O$ (Mr 490.30): C, 46.54:H, 5.26;N,17.14;Cl, 7.23. Found: C, 46.66;H, 5.18; N, 17.04; Cl, 7.28

Example 7 : Preparation of N-[4-[[4-(2,4-Diamino-1,6-dihydro-6-oxo-5-pyrimidinyl)butyl]amino]benzoyl]-(L)-glutamic acid

(a) Preparation of Ethyl 4-[(4-bromobutyl)tosylamino]benzoate

To 0.892 g (0.0372 mol) of sodium hydride resulting from washing 1.11-1.12g of an 80% oil dispersion with hexanes under nitrogen was added 26 mL of dry dimethylformamide. To the stirred mixture was added dropwise over a 21 min period through an addition funnel a solution of 10.00 g (0.0313 mol) of ethyl 4-tosylaminobenzoate (Baker, B.R.; Santi, D.V.; Shapiro, H.S. *J. Pharm Sci.*. **1964**, 53, 1317) in 52 mL of dry dimethylformamide and then 3 mL of rinse dimethylformamide. When the stirred mixture had cooled to room temperature, to it was added at one time 27.74 g (0.128 mol) of 1,4-dibromobutane. The stirred mixture was heated for 4 h 45 min with a bath maintained near 80°C and was allowed to cool to room temperature and stand under nitrogen overnight. The mixture was concentrated under vacuum at 40°C to an oil/solid mixture; yield, 22.37 g. To the mixture was added 100 mL of ethyl acetate, and undissolved solid was collected by suction filtration. The filtrate was purified by a flash chromataography column consisting of 700 g of Silica Gel 60 with 6:1 hexane/ethyl acetate as the eluting solvent. The major fractions of clean product were combined and concentrated under vacuum to a colorless oil that solidified to a white solid; yield, 10.91 g (77%): NMR ($Me_2SOd_6$) δ 1.29 (t, 3H), 1.3-1.5 (m, 2H), 1.7-1.9 (m, 2H), 2.37 (s, 3H), 3.47 (t, 2H), 3.60 (t, 2H), 4.29 (q, 2H), 7.23 (d, 2H), 7.3-7.5 (AA′BB′, 4H), 7.91 (d, 2H). An additional 5% yield was obtained from later column fractions.

(b) Preparation of Ethyl 4-[(5-cyano-6-ethoxy-6-oxohexyl)tosylamino]benzoate

A mixture of 0.506 g (0.0220 mol) of sodium in 10 mL of absolute ethanol under nitrogen was refluxed briefly, and a solution formed. After it had cooled to room temperature, to the solution was added dropwise over a 2-min period 2.74 g (0.0242 mol) of ethyl cyanoacetate. The mixture was refluxed for 10 min and was allowed to cool to room temperature. Then to it was added over a 6-min period 5.00 g (0.0110 mol) of ethyl 4-[(4-bromobutyl)tosylamino]benzoate and 15 mL of rinse absolute ethanol. The mixture was refluxed under nitrogen for 6 h, allowed to cool to room temperature, and stand overnight. It was neutralized with glacial acetic acid and concentrated under vacuum to a wet solid to which was added 50 mL of water and 50 mL of ether. The mixture was shaken in a separatory funnel, and the layers were separated. The aqueous layer was washed with 2x50 mL more ether, and the ether washes were combined, dried over magnesium sulfate for three days, filtered, and concentrated under high vacuum at 60° C to a viscous, pink oil; yield, 5.97 g. The oil was purified by a flash chromatography column consisting of 400 g of Silica Gel 60 with 3:1 hexanes/ethyl acetate as the eluting solvent.

Fractions containing clean product were combined and concentrated under vacuum to a colorless, viscous oil; yield, 2.76 g (50%): NMR ($Me_2SOd_6$) δ 1.19 (t, 3H), 1.29 (t, 3H), 1.3-1.5 (m, 4H), 1.65-1.85 (m, 2H), 2.37 (s, 3H), 3.5-3.7 (m, 2H), 4.14 (t, 1H), 4.15 (q, 2H), 4.29 (q, 2H), 7.22 (d, 2H), 7.3-7.5 (AA′BB′, 4H), 7.90 (d, 2H), 0.15 mol of ethyl acetate.

(c) Preparation of 4-[[4-(2,4-Diamino-1,6-dihydro-6-oxo-5-pyrimidinyl)butyl]tosylamino]benzoic acid

To a solution of 0.377 g (0.0164 mol) of sodium in 8 mL of absolute ethanol under nitrogen was added 1.04 g (0.0109 mol) of guanidine hydrochloride and 1 mL of rinse absolute ethanol. The mixture was stirred for 30 min, and to it was added a solution of 2.73 g (0.00546 mol) of ethyl 4-[(5-cyano-6-ethoxy-6-oxohexyl)-tosylamino]benzoate•0.15 ethyl acetate in 6 mL of absolute ethanol and then 7 mL of rinse absolute ethanol. The mixture was refluxed for 4 h and was allowed to cool to room temperature and stand under nitrogen overnight. The reaction mixture was filtered by suction, and the filtrate was neutralized with 0.984 g (0.0164 mol) of glacial acetic acid. The solution was concentrated under vacuum at 30° C to a solid that was washed well with 50 mL of water, collected by suction filtration, washed again with 25, 25, and 10 mL of water, and dried under vacuum at 60° C; yield, 2.76 g. Fourier transform NMR ($Me_2SOd_6$) and chemical ionization mass spectroscopy indicated a mixture of product and uncyclized intermediates. A 2.41 g sample of solid was added to a solution of 3.16 g (0.137 mol) of sodium in 138 mL of absolute ethanol under nitrogen. The mixture was heated to reflux, and the resulting solution was refluxed for 3 h 15 min. The slightly cloudy solution was allowed to cool to room temperature, and the resulting mixture was allowed to stand under nitrogen overnight. The mixture was heated briefly back to reflux, and the resulting slightly cloudy, hot solution was neutralized with 8.30 g (0.138 mol) of glacial acetic acid. The thick mixture was concentrated under vacuum at 35° C to a white solid to which was added 50 mL of water. Then to the stirred mixture was added approximately 5-8 mL of concentrated hydrochloric acid. The mixture with a pH of 5.5 was filtered by suction, and collected solid was washed with 15 and 20 mL of water and dried under vacuum overnight at room temperature in a desiccator; yield, 1.98 g (84%): NMR ($Me_2SOd_6$) δ 1.2-1.4 (m, 4H), 2.06 (t, 2H), 2.37 (s, 3H), 3.54 (t, 2H), 5.58 (br s, 2H), 5.89 (br s, 2H), 7.14 (d, 2H), 7.3-7.5 (AA′BB′, 4H), 7.86 (d, 2H), 9.75 (br), 0.13 mol of acetic acid.

(d) Preparation of Diethyl N-[4-[[4-(2,4-diamino-1,6-dihydro-6-oxo-5-pyrimidinyl)butyl]tosylamino]benzoyl]-(L)-glutamate

To a stirred mixture of 1.00 g (0.00209 mol) of 4-[[4-(2,4-diamino-1 ,6-dihydro-6-oxo-5-pyrimidinyl)-butyl]tosylamino]benzoic acid•0.13 acetic acid in 6 mL of dry dichloromethane and 6 mL of dry dimethylformamide under nitrogen was added 0.433 g (0.00313 mol) of 1-hydroxybenzotriazole. After 17 min, to the mixture was added 0.646 g (0.00313 mol) of 1,3-dicyclohexylcarbodiimide and then 6 mL more dry dimethylformamide and 6 mL more dry dichloromethane. After 1 h 6 min, to the mixture was added a cloudy solution of 0.750 g (0.00313 mol) of diethyl glutamate hydrochloride and 0.633 g (0.00626 mol) of 4-methylmorpholine in 6 mL of dry dichloromethane and 6 mL of dry dimethylformamide. The mixture was stirred under nitrogen for 59 h 20 min, and to it was added 0.431 g (0.00209 mol) of 1,3-dicyclohexylcarbodiimide. The mixture was stirred for 17 h 15 min and was concentrated under vacuum at 33° C to a residue to which was added 25 mL of methanol. Undissolved white solid was collected by suction filtration, and the filtrate was purified by a flash chromatography column consisting of 175 g of Silica Gel 60 with 8:1 ethyl acetate/methanol as the eluting solvent. Major fractions containing product were combined and

concentrated under vacuum to an off-white solid; yield, 0.74 g (~50%): NMR ($Me_2SOd_6$) δ 1.14 (t, 3H), 1.17 (t, 3H), 1.2-1.4 (m, 4H), 1.9-2.2 (m, 4H), 2.37 (s, 3H) 2.4 (t, 2H), 3.55 (t, 2H), 4.03 (q, 2H), 4.10 (q, 2H), 4.41 (m, 1H), 5.58 (br s, 2H), 5.85 (br s, 2H), 7.15 (d, 2H), 7.3-7.5 (AA′BB′, 4H), 7.81 (d, 2H), 8.75 (d, 1H), 9.68 (br s, 1H), 0.12 mol of dimethylformamide, 0.1 mol of an impurity containing a CONHCH moiety.

(e) Preparation of N-[4-[[4-(2,4-Diamino-1,6-dihydro-6-oxo-5-pyrimidinyl)butyl]amino]benzoyl]-(L)-glutamic acid

A solution of 0.313 g (0.00333 mol) of phenol in 4.70 mL of 31% hydrobromic acid in acetic acid was added to 0.70 g (~0.00105 mol) of diethyl N-[4-[[4-(2,4-diamino-1,6-dihydro-6-oxo-5-pyrimidinyl)butyl]-tosylamino]benzoyl]-L-glutamate •0.12 dimethylformamide•0.1 mol of impurity under nitrogen. A complete solution formed within 20 min. After 1 h 30- min, to the solution was added 39 mL of ether, and a reddish-orange oil formed. The mixture was filtered by suction, and the oil was washed by agitation on the clogged frit with 5x10 mL of ether and dried under vacuum for two days. To the resulting solid/oil mixture was added 15 mL of 1 N sodium hydroxide, and the mixture was stirred under nitrogen in a bath at 40 °C for 1 h 5 min. It was filtered by suction, and the filtrate was acidified to pH 3.5 with concentrated hydrochloric acid. Precipitated solid was collected by suction filtration, washed with 5x5 mL of water, and dried under vacuum overnight; yield, 0.254 g. To a 0.244 g sample was added 10 mL of 1 N sodium hydroxide, and the solution was heated under nitrogen for 30 min in a bath at 40-47 °C and allowed to cool to room temperature. A small amount of solid was removed by suction filtration, and the filtrate was acidified to pH 3.5 with concentrated hydrochloric acid. Precipitated off-white solid was collected by suction filtration, washed with 4x5 mL of water, and dried overnight under vacuum; yield, 0.214 g. A 0.040 g sample of solid wsas dissolved in 20 mL of boiling 95% ethanol, and the solution was treated with 0.005 g of Darco G-60 and filtered. The filtrate was concentrated by boiling to 4 mL, allowed to cool to room temperature, and stand for 1 h. Precipitated pale yellow solid was collected by suction filtration, washed with 2x0.5 mL of 95% ethanol, and dried overnight under vacuum; yield, 0.0135 g (~21%, 90-95% pure by HPLC): NMR ($Me_2SOd_6$) δ 1.2-1.6 (m, 4H), 1.8-2.1 (m, 2H), 2.17 (t, 2H), 2.31 (t, 2H), 2.9-3.1 (m, 2H), 4.2-4.4 (m, 1H), 5.63 (br s, 2H), 5.87 (br s, 2H), 6.19 (t, 1H), 6.52 (d, 2H), 7.62 (d, 2H), 8.04 (d, 1H), 9.75 (br s, 1H), 12.35 (br s, 2H), 0.33 mol of ethanol, weak impurity peaks.

## Chemotherapeutic Data

### A. Cell Culture Method for Evaluation of Compounds as Antitumor Agents

Cells and Medium:

MCF-7 breast adenocarcinoma, obtained from the American Type Culture Collection (ATCC) are grown in RPMI 1640 medium supplemented with 10 nM calcium leucovorin instead of folic acid as the folate source, 10% dialyzed fetal calf serum, penicillin, streptomycin and sodium pyruvate (110 µg/ml).

Cytotoxicity Assay:

Cells are seeded into 96 well plates using a Perkin Elmer Pro/pette. MCF-7 cells are seeded at 15,000 cells per well in 150 µl of medium. Prior to the addition of drugs, cultures were incubated for 24 hours at 37°. Compounds were added at 2x concentration in 150 µl of medium and each concentration was assayed in triplicate. Cultures were incubated for 72 hours in a 37° humidified incubator at 5% $CO_2$. Inhibition of cell growth was measured using the MTT dye reduction assay.

MTT Dye Reduction Assay:

Cell dilutions for a standard curve were prepared from a 72 hour log-phase culture. Serial dilutions were seeded in triplicate in 96 well plates and incubated at 37 for 1 hour. MTT (3-(4,5-dimethylthiazol-2-yl)-2,5-diphenyltetrazolium bromide) was dissolved in PBS at 5 mg/ml and sonicated for 30 seconds. Using the Perkin Elmer Pro/pette, 200 µl of medium was removed and 100 µl of MTT added to the wells of the standard curve and test plates. Suspension cultures were spun for 5 minutes at 1000 rpm before removing medium from the wells. Plates were incubated for 1 hour at 37° on a platform shaker. Following this incubation, 100 µl of medium was removed from the wells and 100 µl of DMSO added to each well. The plates were sonicated for approximately 10 seconds to solubilize the precipitated formazan dye. The absorbance of each well was measured using a Titertek Multiskan MC microtiter plate reader at 570 nm with a reference wavelength of 750 nm.

The compounds of Examples 2, 3, 4, 5 and 6 were found to be active in inhibiting the growth of MCF-7 cells.

## Claims

1.A compound of formula (I):

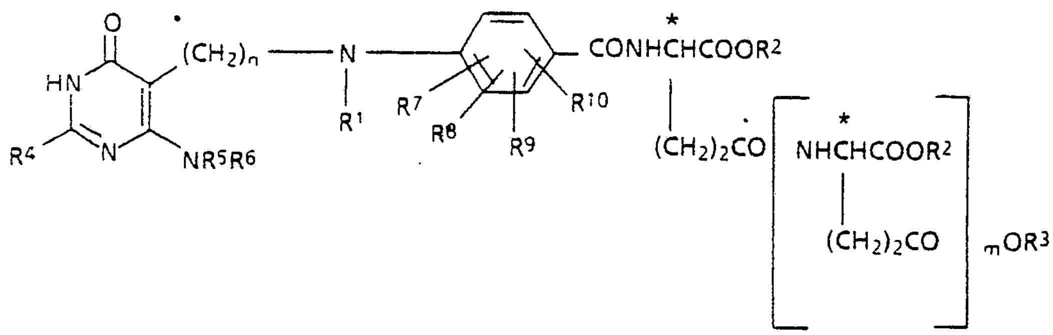

wherein $R^1$ is hydrogen;
$R^2$ and $R^3$ are the same or different and are each hydrogen or $C_{1-4}$ alkyl;
$R^4$ is $NR^{11}R^{12}$;
$R^5$, $R^6$, $R^{11}$ and $R^{12}$ are each hydrogen;
m is zero;
n is 3 and one of $R^7$, $R^8$, $R^9$ and $R^{10}$ is chloro, fluoro, methyl or methoxy and the rest are hydrogen; or n is 4 and each of $R^7$, $R^8$, $R^9$ and $R^{10}$ is hydrogen; and pharmaceutically acceptable salts thereof.

2.A compound according to claim 1 wherein $R^2$ and $R^3$ are both hydrogen.

3.A compound selected from :
N-[4-(3-(2,4-diamino-1,6-dihydro-6-oxo-5-pyrimidinyl)propylamino)-2-fluorobenzoyl]-(L)-glutamic acid,
N-[4-(3-(2,4-diamino-1,6-dihydro-6-oxo-5-pyrimidinyl)propylamino)-3-fluorobenzoyl]-(L)-glutamic acid,
N-[4-(3-(2,4,-diamino-1,6-dihydro-6-oxo-5-pyrimidinyl)propylamino)-3-methylbenzoyl]-(L)-glutamic acid,
N-[4-(3-(2,4-diamino-1 ,6-dihydro-6-oxo-5-pyrimidinyl)propylamino)-3-(methoxy)benzoyl]-(L)-glutamic acid,
N-[4-(3-(2,4-diamino-1 ,6-dihydro-6-oxo-5-pyrimidinyl) propylamino)-2-chlorobenzoyl]-(L)-glutamic acid, and
N-[4[[4-(2,4-diamino-1,6-dihydro-6-oxo-5-pyrimidinyl)butyl]amino]benzoyl] -(L)-glutamic acid,
or a pharmaceutically acceptable salt thereof.

4.A pharmaceutical formulation which comprises a compound according to any of claims 1 to 3 or a pharmaceutically acceptable salt thereof and a pharmaceutically acceptable carrier therefor.

5.A method for preparing a pharmaceutical formulation which comprises bringing into association a compound according to any of claims 1 to 3 or a pharmaceutically acceptable salt thereof with a pharmaceutically acceptable carrier therefor.

6 A compound as defined in any of claims 1 to 3 for use as a medicament.

7. Use of a compound as defined in any of claims 1 to 3 for the manufacture of a medicament for the treatment of neoplastic growth.

8.A process for the preparation of a compound of formula (I), as defined herein, or a salt thereof, which comprises deacylating a compound of formula (II):

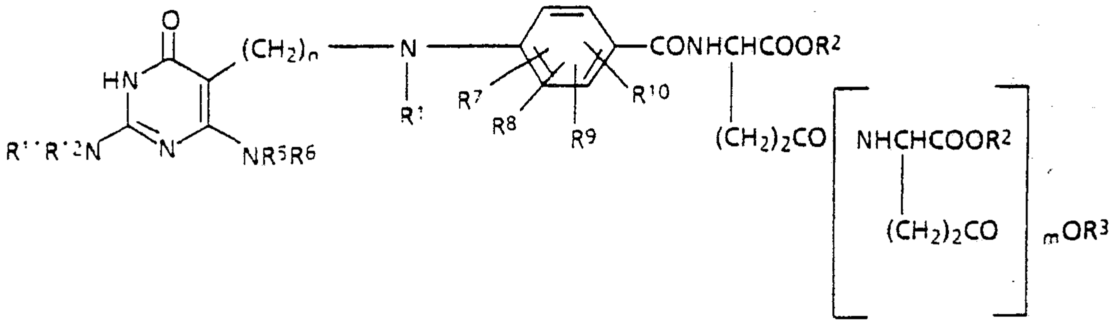

wherein $R^1$, $R^2$, $R^3$, $R^7$, $R^8$, $R^9$ and $R^{10}$ and n and m are as defined above in formula (I); one of the groups $R^5$, $R^6$, $R^{11}$ and $R^{12}$ is $C_{1-12}$ acyl and the other groups $R^5$, $R^6$, $R^{11}$ and $R^{12}$ are the same or different and are hydrogen or $C_{1-12}$ acyl, and optionally thereafter converting one or both of $R^2$ and $R^3$ in the resulting compound of formula (I) into a different $R^2$ and/or $R^3$, and optionally forming a salt.

Claims for the following Contracting States: ES,GR

1.A process for the preparation of a compound of formula (I):

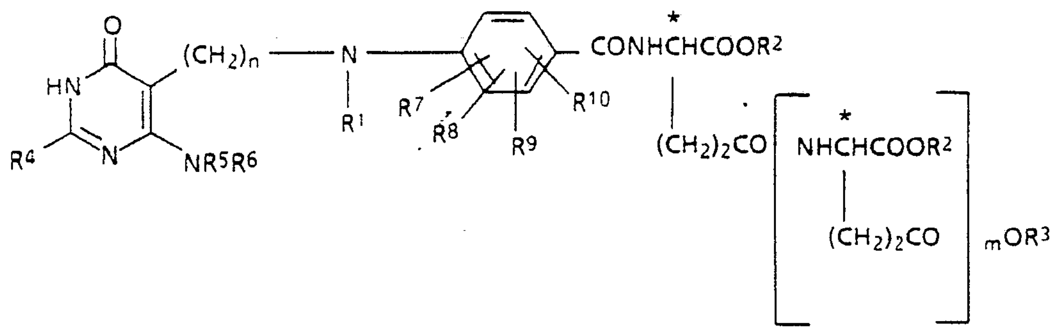

wherein $R^1$ is hydrogen;
$R^2$ and $R^3$ are the same or different and are each hydrogen or $C_{1-4}$ alkyl;
$R^4$ is $NR^{11}R^{12}$;
$R^5$, $R^6$, $R^{11}$ and $R^{12}$ are each hydrogen;
m is zero;
n is 3 and one of $R^7$, $R^8$, $R^9$ and $R^{10}$ is chloro, fluoro, methyl or methoxy and the rest are hydrogen;
or n is 4 and each of $R^7$, $R^8$, $R^9$ and $R^{10}$ is hydrogen;
and pharmaceutically acceptable salts thereof, a process for the preparation of a compound of formula (I), as defined herein, or a salt thereof, which comprises deacylating a compound of formula (II):

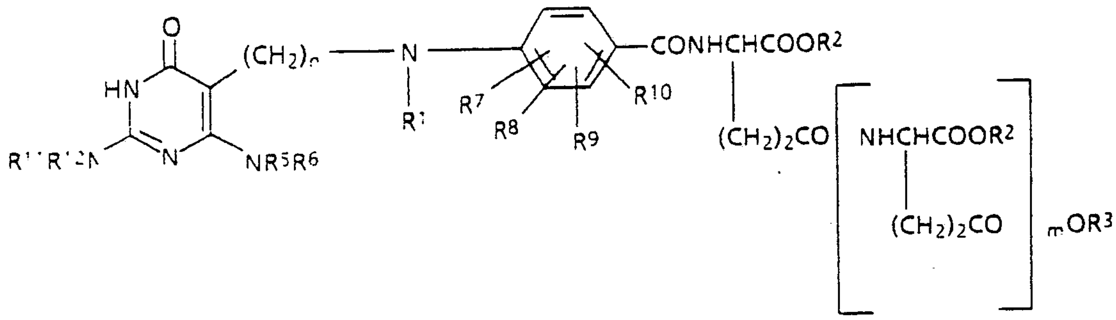

wherein $R^1$, $R^2$, $R^3$, $R^7$, $R^8$, $R^9$ and $R^{10}$ and n and m are as defined above in formula (I); one of the groups $R^5$, $R^6$, $R^{11}$ and $R^{12}$ is $C_{1-12}$ acyl and the other groups $R^5$, $R^6$, $R^{11}$ and $R^{12}$ are the same or different and are hydrogen or $C_{1-12}$ acyl, and optionally thereafter converting one or both of $R^2$ and $R^3$ in the resulting

compound of formula (I) into a different $R^2$ and/or $R^3$, and optionally forming a salt.

2.A process according to claim 1 wherein $R^2$ and $R^3$ are both hydrogen.

3.A process according to claim 1 for preparing a compound selected from :
N-[4-(3-(2,4-diamino-1 ,6-dihydro-6-oxo-5-pyrimidinyl)propylamino)-2-fluorobenzoyl]-(L)-glutamic acid,
N-[4-(3-(2,4-diamino-1,6-dihydro-6-oxo-5-pyrimidinyl)propylamino)-3-fluorobenzoyl]-(L)-glutamic acid,
N-[4-(3-(2,4,-diamino-1,6-dihydro-6-oxo-5-pyrimidinyl)propylamino)-3-methylbenzoyl]-(L)-glutamic acid,
N-[4-(3-(2,4-diamino-1,6-dihydro-6-oxo-5-pyrimidinyl)propylamino)-3-(methoxy)benzoyl]-(L)-glutamic acid,
N-[4-(3-(2,4-diamino-1 ,6-dihydro-6-oxo-5-pyrimidinyl)propylamino)-2-chlorobenzoyl]-(L)-glutamic acid, and
N-[4[[4-(2,4-diamino-1 ,6-dihydro-6-oxo-5-pyrimidinyl)butyl]amino]benzoyl] -(L)-glutamic acid,
or a pharmaceutically acceptable salt thereof.

4.A pharmaceutical formulation which comprises a compound according to any of claims 1 to 3 or a pharmaceutically acceptable salt thereof and a pharmaceutically acceptable carrier therefor.

5.A method for preparing a pharmaceutical formulation which comprises bringing into association a compound according to any of claims 1 to 3 or a pharmaceutically acceptable salt thereof with a pharmaceutically acceptable carrier therefor.

6 A compound as defined in any of claims 1 to 3 for use as a medicament.

7.Use of a compound as defined in any of claims 1 to 3 for the manufacture of a medicament for the treatment of neoplastic growth.